# EUROPEAN PATENT SPECIFICATION

(11) **EP 1 615 871 B1**
(45) Date of publication and mention of the grant of the patent: **29.10.2008**
(21) Application number: 04727059.0
(22) Date of filing: 13.04.2004
(51) Int. Cl.: C07C 67/00, C07C 69/716

(54) **PROCESS FOR THE PREPARATION OF DIALKYL 3-OXOGLUTARATES**
VERFAHREN ZUR HERSTELLUNG VON DIALKYL 3-OXOGLUTARATEN
PROCEDE DE PREPARATION DE DIALKYL-3-OXOGLUTARATES

(30) Priority: 10.04.2003 HU 0300941; 04.02.2004 HU 0400351
(43) Date of publication of application: 18.01.2006
(73) Proprietor: EGIS Gyógyszergyár Nyilvánosan Müködõ Részvénytársaság, 1106 Budapest (HU)
(72) Inventor: JAKFALVI, Elemér, H-1162 Budapest (HU); GREGORNE BOROS, Livia, H-2141 Csömör (HU); MORASZ, Tamás, H-1163 Budapest (HU); TOTH, Gábor, H-2081 Piliscsaba (HU); SZABO , Attila, H1196 Budapest (HU); TÖREKI, József, H-2143 Kistarcsa (HU); OLAH, Sándor, H-2040 Budaörs (HU)
(74) Representative: Hirsch & Associés
(86) International application number: PCT/HU2004/000035
(87) International publication number: WO 2004/089867

(56) References cited:
- GB-A- 2 033 391

## Description

### Field of the invention

This invention relates to a process for the preparation of dialkyl 3-oxoglutarates ensuring the production of these compounds in an easily feasible way on an industrial scale and resulting in high-purity products.

### Technical background of the invention

Dimethyl 3-oxoglutarate (that is the compound of the general formula (I), 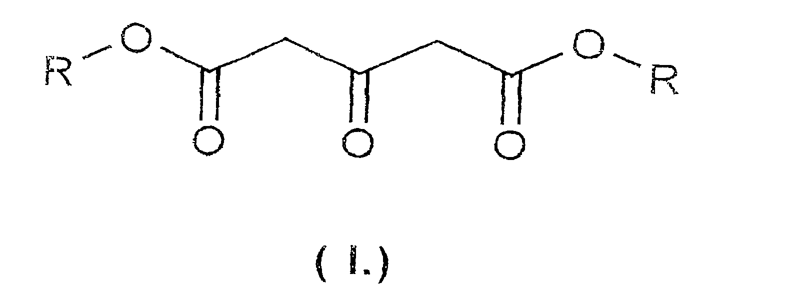 wherein R stands for methyl) is a valuable pharmaceutical intermediate.

A high number of processes are known from the literature for the preparation of the compounds of the general formula (I).

The published European patent application No. 47,514 discloses the preparation of the corresponding ethyl ester (diethyl 3-oxoglutarate) starting from ethyl chloroacetoacetate. The starting substance is carbonylated in an ethanol medium, in the presence of potassium carbonate and dicobalt octacarbonyl; the reaction mixture is heated under a carbon monoxide atmosphere and under a pressure of 100 bar for 7 hours at a temperature of 65 °C to obtain diethyl 3-oxobutarate in a yield of 60 %. This process, however, is less feasible on an industrial scale, because dicobalt octacarbonyl is extremely dangerous and liable to decomposition.

According to the US patent specification No. 3,963,775 dimethyl 3-oxoglutarate is prepared by reacting ketene, phosgene and methanol. The yield of this process is about 50%. The drawbacks of this method are the poor yield on the one hand and the application of phosgene, this extremely poisonous compound on the other hand. This process is not recommended for an industrial scale production either.

According to the published European patent application No. 108,332 and the Japanese patent specification No. 60/120838 diesters of acetone-carboxylic acid are prepared by the reaction of diketene, carbon monoxide and nitrite esters carried out in the presence of palladium halides and copper salts. Dimethyl 3-oxoglutarate is prepared in a yield of about 60% by reacting diketene, carbon monoxide and methyl nitrite in the presence of palladium chloride and copper(II) chloride. The reaction is carried out at a temperature of 60 °C for 3 hours. The drawbacks of this process reside in the moderate yield and the application of diketene, which substance is disadvantageous when used in an industrial scale production.

According to the publication disclosed in Chem. Pharm. Bull. 29, (10), 2762-2768 (1981) the monomethyl ester of malonic acid is reacted with 1,1-carbodiimidazole, and the thus-obtained intermediate is treated with diluted hydrochloric acid. The drawback of this process is that the applied 1,1-carbodiimidazole is a very expensive reagent, it is nearly four times more expensive than dimethyl 3-oxoglutarate.

The publication Recl. Trav. Chim. Pays-Bas, 108, (2) 51-56 (1989) discloses a process wherein by starting from methyl acetoacetate a salt is formed with lithium diisopropyl amide in tetrahydrofurane at a temperature of -45 °C, which salt is then converted into dimethyl 3-oxoglutarate by alkylation carried out with dimethyl carbonate.

According to the Swiss patent specification No. 659,060 methyl acetoacetate is applied as starting material, which is converted into an enamine with a secondary amine (such as pyrrolidone). The thus-obtained enamine is then reacted with sodium amide in liquid ammonia, the anion thus obtained is alkylated with methyl chloroformiate, and the desired compound is then obtained by hydrolysis performed with diluted hydrochloric acid.

The drawback of the two latter methods resides in the fact that the applied reagents and the required reaction temperature (reaction with lithium diisopropyl amide carried out at a temperature of -45 °C and the application of sodium amide and liquid ammonia) require special measures cumbersome under industrial circumstances (such as a perfect inertization, exclusion of traces of humidity, intensive cooling performed e.g. by the application of a cooling medium consisting of a mixture of dry-ice and acetone).

According to the publication Org. Synth. Coll. Vol. I. 10, 237 (1946) acetone dicarboxylic acid is prepared first from citric acid monohydrate in fuming sulphuric acid, which is then converted into the corresponding diester in anhydrous ethanol, in the presence of sulphuric acid catalyst, in a yield of about 40 %. This method has several drawbacks. The yield is rather poor, the application of a high amount of fuming sulphuric acid is necessary and the acetone dicarboxylic acid formed as an intermediate, a compound liable to decomposition, is to be isolated.

A method similar to the above-described synthesis, which is, however, somewhat more effective, is described by Findley, S.P. in J. Org. Chem. 22, 1385 (1957). According to this method anhydrous citric acid is converted into acetone dicarboxylic acid in fuming sulphuric acid, which is then esterified in situ with anhydrous methanol. Dimethyl 3-oxoglutarate is obtained after isolation and distillation in a yield of 64%. The drawback of this process is that it ensures the product only in a moderate yield even in an experimental scale.

According to the method disclosed in Indian J. Chem. Sect. B 37, (4), 397-398 (1998) a process similar to the one mentioned in the latter reference is carried out, with the difference that instead of fuming sulphuric acid concentrated sulphuric acid is reacted with citric acid monohydrate, and the thus-obtained acetone dicarboxylic acid is esterified in situ with methanol. The crude oil is subjected to distillation in vacuo to obtain dimethyl 3-oxoglutarate in a yield of 52%. The aim of the authors was to elaborate a synthesis suitable for a larger scale production than those known from the earlier publications. This method, however, ensures only a medium yield even when using the starting materials in an order of magnitude of 100-200 g.

The German Patent specification No. 1,092,900 reports the application of the process disclosed in the above publication by using quantities of about 100 kg of the starting materials. Crude dimethyl 3-oxoglutarate is obtained only in a yield of 58%.

According to the German patent specification No. 1,160,841 in a laboratory scale production citric acid is reacted first with chlorosulphonic acid instead of sulphuric acid or fuming sulphuric acid, then with methanol. The crude dimethyl 3-oxoglutarate is obtained by distillation in vacuo in a yield of 90%.

In the processes specified above the applied sulphuric acid, fuming sulphuric acid and chlorosulphonic acid act both as reagents and as solvents in the stage of the formation of acetone dicarboxylic acid. This is, however, disadvantageous in an industrial scale production, since a considerable amount of non-regenerable waste acid is formed during the reaction. The processing of waste acid (neutralization, etc.) with respect to environmental protection requirements needs considerable expenses. A further disadvantage of the processes performed without applying solvents is that due to the vigorous gas formation (carbon monoxide is liberated during the reaction) it is very difficult to observe the temperature range, so in an industrial scale production it is problematical to keep the reaction under control.

The process provided in the Belgian patent specification No. 879,537 aims at eliminating the above-mentioned drawback. Into methylene chloride solvent first chlorosulphonic acid is measured, then citric acid is added to it. The intermediate thus obtained is esterified with methanol, the desired product is isolated, thus dimethyl 3-oxoglutarate is obtained in a yield of 94 to 96%.

The above-mentioned Belgian patent application was filed in 1979. During the more than two decades passed since then the requirements in the field of pharmaceutical industry have become considerably stricter. Pharmacopoeias usually allow the presence of an amount of at most 0.1 % of identified or unidentified impurities in the final product and a total impurity content of not exceeding 0,5%. Consequently, requirements towards intermediates are also stricter and stricter, as impurities of the intermediates may get into the final product. Besides, they may lead to the formation of further impurities by undesired side-reactions.

When reproducing the process described in the Belgian patent specification No. 879,537 we have found that if the reaction is carried out under the conditions specified in the examples, a lot of side-reactions occur. The removal of the side-products formed in considerable amounts is an expensive procedure requiring additional operations and, on the other hand, it is difficult to separate the impurities due to the similar physical properties of the side-products. There is therefore a need for a process resulting in dimethyl 3-oxoglutarate in a purity meeting the up-to-date requirements of the pharmaceutical industry, that is containing side-products in an amount smaller than that prepared by the hitherto known methods.

The aim of the invention was to elaborate a method for the preparation of dialkyl 3-oxoglutarates of the general formula (I), wherein R stands for methyl or ethyl, which meets the above requirements and provides these compounds in high purity, containing only a small amount of side-products. Now it has been found that this aim can be achieved by the process of the present invention.

### Summary of the invention

According to the present invention there is provided a process for the preparation of 3-oxoglutarates of the general formula (I), wherein R stands for methyl or ethyl. Said process comprises reacting citric acid of the formula (II) 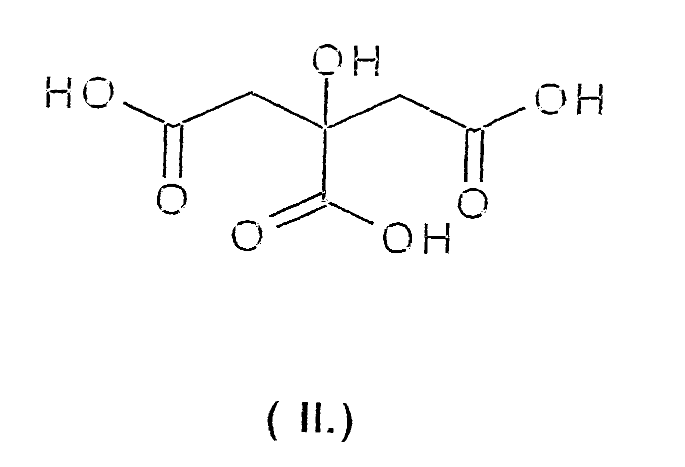 or the monohydrate thereof with chlorosulphonic acid in a lower chlorinated aliphatic hydrocarbon and reacting the intermediate thus obtained with methanol or ethanol, which comprises applying a ratio of lower chlorinated aliphatic hydrocarbon to chlorosulphonic acid in the range of 0,1:1 to 1:1 by volume and carrying out the addition of citric acid to the mixture of chlorosulphonic acid and lower aliphatic hydrocarbon at a rate of 1 - 2 kg/minute.

### Description of the preferred embodiments

According to a preferred embodiment of the process according to the invention dimethyl 3-oxoglutarate, that is the compound of the general formula (I), wherein R is a methyl group, is prepared.

As reaction medium a lower chlorinated aliphatic hydrocarbon, such as methylene chloride, ethylene chloride or chloroform, preferably methylene chloride or ethylene chloride, particularly methylene chloride may be used.

The reaction temperature is preferably kept between 10 °C and 15 °C during the addition of the citric acid until the gas formation has been ceased.

When studying the process for the preparation of dimethyl 3-oxoglutarate we have established that the following side-reaction may occur:

In the method illustrated by reaction scheme 1 dimethyl 3-oxoglutarate is present also in the form of "enol" in an equilibrium process. When abstracting water, dimethyl 3-methoxy-pentenedioate is formed either in an indirect manner via the "ketene" compound obtained in an acidic medium, or directly, upon using methanol as esterifying agent. This "enol ether" cannot be separated from the dimethyl 3-oxoglutarate by vacuum distillation due to the small difference in the boiling points, so it remains in the main evaporate together with the dimethyl 3-oxoglutarate. The amount of "enol ether" may be as high as 4 - 5%.

In the method illustrated by reaction scheme 2 trimethyl citrate is formed from citric acid in acidic medium in the presence of methanol, which contaminates the desired dimethyl 3-oxoglutarate in an amount of 2 - 3%.

In the method illustrated by reaction scheme 3 trimethyl aconitate contamination is formed in an amount of 0.4 - 1.0% from citric acid (without decarbonylation) by the elimination of one molecule of water and the subsequent esterification. Due to the small difference in the boiling points the trimethyl aconitate cannot be separated from the dimethyl 3-oxoglutarate by distillation.

The invention is based on the recognition that the above contaminations, when producing dimethyl 3-oxoglutarate by the process according to the invention, are formed in considerably smaller amounts.

Surprisingly it has been found that the formation of dimethyl 3-methoxypentenedioate (enol ether) in the reaction of citric acid with chlorosulphonic acid carried out in a lower chlorinated aliphatic hydrocarbon medium depends on the ratio of the lower chlorinated aliphatic hydrocarbon to chlorosulphonic acid. Upon decreasing the amount of solvent related to the amount of chlorosulphonic acid the undesired "enol ether" formation can be reduced. In the process according to the invention the ratio by volume of the lower chlorinated aliphatic hydrocarbon to chlorosulphonic acid is in the range of 0.1:1 to 1:1, preferably 0.4:1 to 0.6:1, particularly 0.5:1. As it can be seen from Example 1, when applying the above volume ratio the enol ether content of the prepared final product is 0.81%. On the other hand, when applying a ratio by volume of methylene chloride to chlorosulphonic acid of 2.83:1 as described in Example 1 of the Belgian patent specification No. 879,537, the obtained dimethyl 3-oxoglutarate contains 4.78% of enol ether.

The invention is also based on the further recognition that the formation of the undesired trimethyl citrate contamination can be reduced by decreasing the rate of addition of the citric acid. Namely, citric acid does not dissolve in the applied reaction medium, so upon adding it rapidly small lumps are formed, which do not enter into reaction with chlorosulphonic acid, but in a later stage of the reaction form trimethyl citrate with the methanol applied as an esterifying agent.

According to the method of this invention the addition of citric acid to the mixture of chlorosulphonic acid and lower chlorinated aliphatic hydrocarbon is carried out at a rate in the range of 1 to 2 kg/minute, preferably 1.1 to 1.8 kg/minute, especially 1.25 to 1.5 kg/minute. According to Example 1 illustrating the method of this invention the final product contains 0,09% of trimethyl citrate. On the other hand, when reproducing the method disclosed in the Belgian patent specification No. 879,537 (comparative Example 2) and applying methylene cloride and chlorosulfonic acid in a ratio by volume of 2.83:1, the obtained dimethyl 3-oxoglutarate contains 2.27 % of trimethyl citrate.

Furthermore, the invention is based on the recognition that by applying the method according to the invention the formation of the trimethyl aconitate side-product can also be decreased. The product prepared according to Example 1 contains 0.28% of trimethyl aconitate. On the other hand, when dimethyl 3-oxoglutarate is prepared according to example 1 of the Belgian patent specification No. 879,537, it contains 0.46% of trimethyl aconitate contamination.

It has also been recognised that the contamination content of the product prepared according to the invention is lower if the reaction temperature is kept between 10 °C and 15 °C during the addition of the citric acid and the subsequent gas formation.

Comparative analytical data reveal that the product prepared according to this invention is of considerably higher purity than that produced by the known method. According to Example 1 of this invention the dimethyl 3-oxoglutarate content is 98.33%, while by proceeding as specified in Example 2, which is a reproduction of the method described in the Belgian patent specification No. 879,537, a purity of only 91.07% can be achieved.

The process according to the invention is preferably carried out as follows:

A 0.5:1 mixture of a lower chlorinated aliphatic hydrocarbon and chlorosulphonic acid is prepared. An anhydrous solvent is to be used for the process. The temperature of the mixture is adjusted to a temperature between 15 °C and 20 °C. Then citric acid is added to it at a rate of 1 to 2 kg/minute. For this purpose anhydrous citric acid or - preferably - citric acid monohydrate is applied. During the addition the temperature is kept at a temperature between 18 °C and 22 °C, which can be easily achieved by carrying out the addition at a rate specified above. The reaction mixture is then stirred at a temperature between 20 °C and 22 °C until the gas formation has been ceased. This operation takes at least 6 hours, preferably 7 to 8 hours.

The reaction mixture is then cooled to 3 to 5 °C and methanol or ethanol is added to it. The addition usually takes about 4 to 6 hours. Methanol or ethanol is added at such a rate that the inner temperature do not exceed 25 °C. When all the methanol or ethanol has been added, the mixture is warmed to a temperature between 30 °C and 35 °C and stirred for about 1 to 3 hour(s) in order to complete the esterification reaction. When it has been completed, the mixture is cooled to a temperature between 10 °C and 12 °C.

To the thus-obtained mixture water is added carefully at such a rate that the temperature remains below 15 °C. Then methylene chloride is introduced to it. The mixture is stirred, and then clarified in order to separate the phases. The organic phase is separated. The aqueous phase is extracted, with methylene chloride. The organic phases are combined, washed first with water than with a basic solution - preferably an aqueous alkali hydrocarbonate solution. The pH value of the aqueous phase is adjusted to about 7 by the washing operation. The organic phase is separated, washed with water and evaporated in vacuo. By using this method dimethyl 3-oxoglutarate can be obtained in an excellent yield even on an industrial scale and in such a purity that the product can be used for the further reaction steps without further purification.

The advantages of the process according to the invention are as follows:
- it can be carried out without applying explosive and expensive reagents;
- it can be performed readily and to advantage even on an industrial scale;
- the final product contains much less side-products than those prepared according to the known methods;
- it can be carried out in an excellent yield of higher than 95 %.

Further details of the present invention are to be found in the following Examples, without limiting the scope of protection to these Examples.

### Example 1

490 kg (370 1) of anhydrous methylene chloride are added to 1320 kg (753 1) of chlorosulphonic acid (the ratio of methylene chloride to chlorosulphonic acid is 0.5:1 by volume). The temperature of the mixture is adjusted to 10 - 15 °C, and 665.0 kg of citric acid monohydrate are added to it at a rate of 1.25 kg/minute, while the temperature of the reaction mixture is kept between 10 °C and 15 °C. When the addition has been completed the reaction mixture is stirred at a temperature between 10°C and 15°C until no more gas is liberated (at least 6 hours).

The reaction mixture is then cooled to 3 - 5 °C and 640 kg (800 1) of anhydrous methanol are added to it at such a rate that the inner temperature does not exceed 25°C. The reaction mixture is then warmed to 30 - 35°C and stirred at the same temperature for 2 hours. The esterifying reaction is completed under this period.

The reaction mixture is cooled to 10 - 12 °C and 1300 1 of water are added to it at such a rate that the temperature does not exceed 15 °C . To the mixture 800 ml of methylene chloride are added and it is stirred for 15 minutes. The two-phase mixture is clarified for 30 minutes and the organic phase is separated. The aqueous layer is extracted three times with 400 ml each of methylene chloride. The organic phases are combined and washed first with 1000 1 of water, then with a solution of 75 kg of sodium hydrocarbonate in 1000 1 of water. The pH of the solution is adjusted to a value of about 7 by the addition of sodium hydrocarbonate. The organic phase is separated, washed twice with 750 ml each of water and evaporated in vacuo. Evaporation is carried out under a pressure of 3 - 5 kPa until the inner temperature rises at most to 75 °C. Thus 535 kg of dimethyl 3-oxoglutarate are obtained, which can be used for the further reaction steps without purification.

Qualification by gas chromatography:
Purity by gas chromatography:
Apparatus: gas chromatograph of HP- 6850 type
Method: Column: HP- 1.25 m x 0.32 mm, 0.17 µm of film thickness
Carrier gas: hydrogen
Program: 80 °C - 5 minutes - 10 °C/minute - 200 °C - 8 minutes
Detector: FID 280 °C
Injector: 200 °C.
Expected retention times:

| | |
|---|---|
| Methyl acetoacetate | 1.18 minutes |
| Dimethyl 3-oxoglutarate | 5.77 minutes |
| Enol ether | 8.15 minutes |
| Trimethyl aconitate | 10.00 minutes |
| Trimethyl citrate Isoftalic acid | 10.42 minutes |
| derivative | 18.76 minutes |

Evaluation: by area normalization
Result:

| | |
|---|---|
| Methyl acetoacetate: | 0.31% |
| Dimethyl 3-oxoglutarate: | 98.33% |
| Enol ether: | 0.81% |
| Trimethyl aconitate: | 0.28% |

| | |
|---|---|
| Trimethyl citrate: | 0.09% |
| Isoftalic acid derivative: | - |
| Other contamination | |
| (higher than 0,2 %) | - |

### Example 2

Into a mixture of 100.0 g (58 ml) of chlorosulphonic acid and 30 ml of methylene chloride 50.0 g of citric acid monohydrate are added within 30 minutes at a temperature between 10 °C and 15 °C. The mixture is stirred at the same temperature for 5 hours, cooled to 3 - 5 °C and 60 ml of methanol are introduced to it within 15 minutes at such a rate that the temperature does not exceed 25 °C. It is then stirred for 2 hours at a temperature between 30 °C and 35 °C, cooled to 15 °C and 100 ml of water are added to it within 15 minutes. The thus-obtained mixture is extracted three times with 50 ml each of methylene chloride; the organic phases are combined, washed successively with 100 ml of saturated sodium hydrogen carbonate solution and 100 ml of water and evaporated in vacuo. Thus 40.8 g of dimethyl 3-oxoglutarate are obtained.
Yield: 98.5 %
Apparatus: gas chromatograph of HP-6850 type
Method: Column: Hop-1.25 m x 0.52 mm, film thickness of 0.17 µm
Carrier gas: hydrogen
Program: 80 °C - 5 minutes - 10 °C/minute - 200 °C - 8 minutes

| | |
|---|---|
| Detector: FID 280 °C | |
| Injector: 200 °C. | |
| Expected retention | times: |
| Methyl acetoacetate | 1.18 minutes |
| Dimethyl 3-oxy-glutarate | 5.77 minutes |
| Enol ether | 8.15 minutes |
| Trimethyl citrate | 10.42 minutes |
| Trimethyl aconitate | 10.11 minutes |
| Isoftalic acid | |
| derivative | 18.76 minutes |
| Evaluation: by area | normalization |

Evaluation by gas chromatography (the method was carried out using the parameters specified in Example 1) :
Result:

| | |
|---|---|
| Methyl acetoacetate: | 0.49% |
| Dimethyl 3-oxoglutarate: | 97.62% |
| Enol ether: | 0.98% |
| Trimethyl citrate: | 0.13% |
| Trimethyl aconitate: | 0.29% |
| Isoftalic acid derivative: | 0.07% |
| Other contamination | |
| (more than 0.2 %) | |

### Example 3 (comparative example)

Example 1 of the Belgian patent specification No. 879,537

A mixture of 212 ml of chlorosulphonic acid and 600 ml of methylene chloride is prepared (the ratio of methylene chloride to chlorosulphonic acid is 2.83:1 by volume). Then 200.0 g of citric acid are added to this mixture within 15 - 20 minutes, the addition rate of citric acid is 13.3 kg/minute, temperature = 20 - 22 °C. The reaction mixture is stirred at this temperature for 5 hours, cooled to a temperature between 3 °C and 5 °C and 320 ml of methanol are added to it within 15 minutes at such a rate that the temperature remain below 25 °C. The reaction mixture is then stirred for 2 hours at a temperature between 30 °C and 35 °C, cooled to 15 °C, poured into 800 ml of water, stirred vigorously for 15 minutes, clarified for 15 minutes and the phases are separated. The aqueous phase is extracted three times with 150 ml each of methylene chloride. The organic phases are combined, washed once with 400 ml of saturated sodium hydrogen carbonate solution and twice with 200 ml each of water and evaporated in vacuo. Thus 158.2 g of dimethyl 3-oxoglutarate are obtained. Yield: 87.3 %.
Evaluation by gas chromatography (the method was carried out using the parameters specified in Example 1) :
Result:

| | |
|---|---|
| Methyl acetoacetate: | 0.21% |
| Dimethyl 3-oxoglutarate: | 91.07% |
| Enol ether: | 4.78% |
| Trimethyl aconitate | 0.46% |
| Trimethyl citrate: | 2.27% |
| Isoftalic acid derivative: | 0.53% |
| Other contamination | |
| (higher than 0.2%) | - |

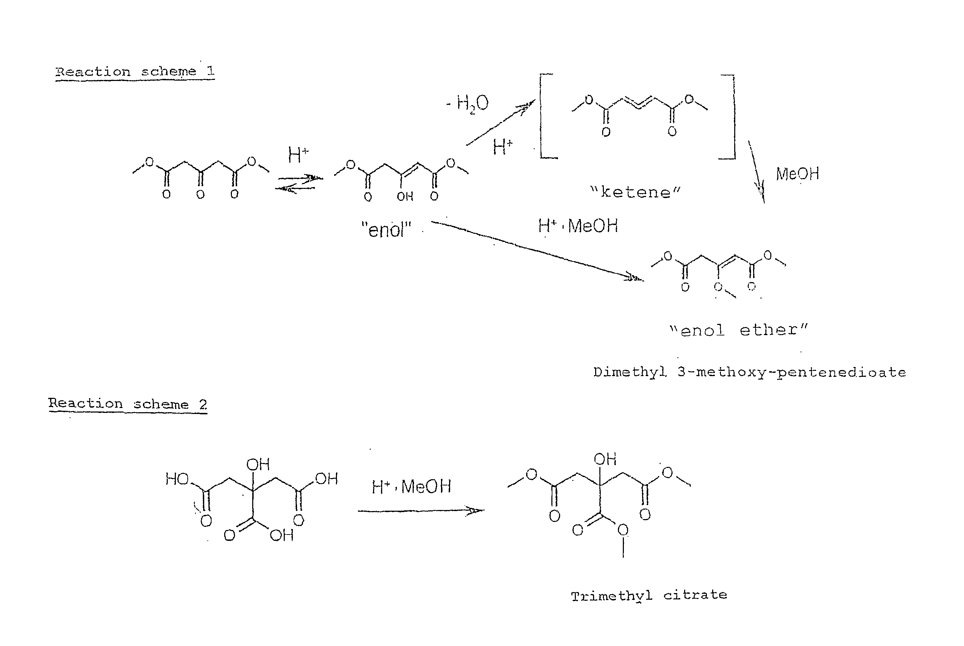

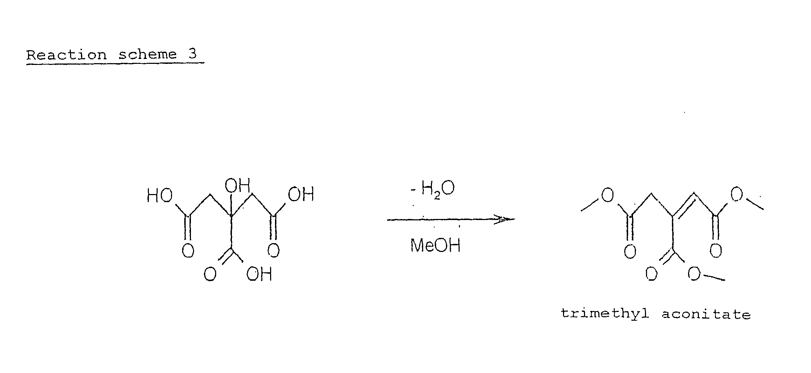

## Claims

1. A process for the preparation of dialkyl 3-oxoglutarates of the general formula (I), 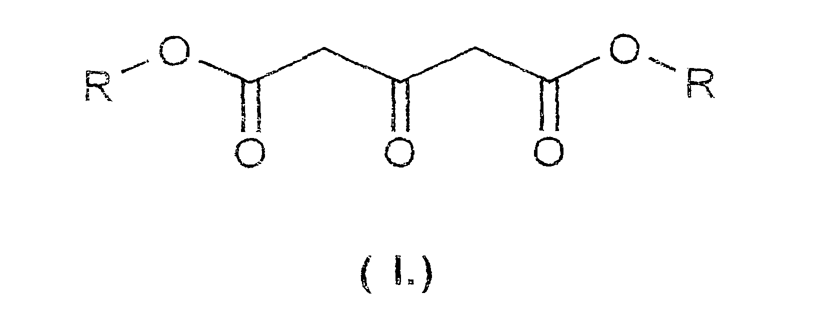 wherein R stands for methyl or ethyl, by reacting citric acid of the formula (II) 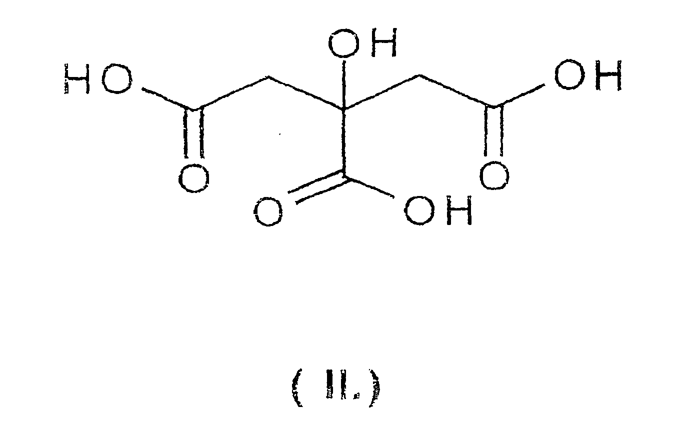 or the monohydrate thereof with chlorosulphonic acid in a lower chlorinated aliphatic hydrocarbon and reacting the intermediate thus obtained with methanol or ethanol, which comprises applying a ratio of lower chlorinated aliphatic hydrocarbon to chlorosulphonic acid in the range of 0,1:1 to 1:1 by volume and carrying out the addition of citric acid to the mixture of chlorosulphonic acid and lower aliphatic hydrocarbon at a rate of 1 - 2 kg/minute.

2. A process as claimed in claim 1, which comprises using as lower aliphatic hydrocarbon methylene chloride, ethylene chloride or chloroform.

3. A process as claimed in claim 2, which comprises using methylene chloride.

4. A process as claimed in claim 3, which comprises using methylene chloride and chlorosulphonic acid in a volume ratio in the range of 0.4:1 to 0.6:1.

5. A process as claimed in claim 4, which comprises using methylene chloride and chlorosulphonic acid in a volume ratio of 0.5:1.

6. A process was claimed in any of claims 1 to 5, which comprises carrying out the addition of citric acid to the mixture of chlorosulphonic acid and lower chlorinated aliphatic hydrocarbon at a rate in the range of 1.1 to 1.8 kg/minute.

7. A process as claimed in claim 6, which comprises carrying out the addition of citric acid to the mixture of chlorosulphonic acid and lower chlorinated aliphatic hydrocarbon at a rate in the range of 1.25 to 1.5 kg/minute

8. A process as claimed in any of claims 1 to 7, which comprises keeping the reaction temperature during the addition of the citric acid and the subsequent gas formation between 10 °C and 15 °C.

## Patentansprüche

1. Verfahren zur Herstellung von Dialkyl-3-oxoglutaraten der allgemeinen Formel (I) 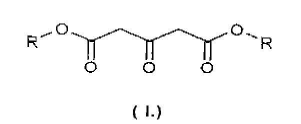 wobei R für Methyl oder Ethyl steht, indem Citronensäure der Formel (II) 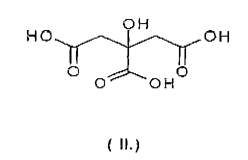 oder das Monohydrat davon mit Chlorschwefelsäure zu einem niederen chlorierten aliphatischen Kohlenwasserstoff umgesetzt wird, und indem das so erhaltende Zwischenprodukt mit Methanol oder Ethanol umgesetzt wird, das das Anwenden eines Verhältnisses von niederem chloriertem aliphatischem Kohlenwasserstoff zu Chlorschwefelsäure im Bereich von 0,1:1 bis 1:1 Volumenteilen umfasst und das Durchführen der Zugabe von Citronensäure zu dem Gemisch aus Chlorschwefelsäure und niederem aliphatischem Kohlenwasserstoff mit einer Geschwindigkeit von 1 - 2 kg/Minute.

2. Verfahren nach Anspruch 1, das die Verwendung von Methylenchlorid, Ethylenchlorid oder Chloroform als niedrigem aliphatischem Kohlenwasserstoff umfasst.

3. Verfahren nach Anspruch 2, das die Verwendung von Methylenchlorid umfasst.

4. Verfahren nach Anspruch 3, das die Verwendung von Methylenchlorid und Chlorschwefelsäure in einem Volumenverhältnis im Bereich von 0,4:1 bis 0,6:1 umfasst.

5. Verfahren nach Anspruch 4, das die Verwendung von Methylenchlorid und Chlorschwefelsäure in einem Volumenverhältnis von 0,5:1 umfasst.

6. Verfahren nach einem der Ansprüche 1 bis 5, das das Durchführen der Zugabe von Citronensäure zu dem Gemisch aus Chlorschwefelsäure und niedrigem chloriertem aliphatischem Kohlenwasserstoff mit einer Geschwindigkeit im Bereich 1,1 bis 1,8 kg/Minute umfasst.

7. Verfahren nach Anspruch 6, das das Durchführen der Zugabe von Citronensäure zu dem Gemisch aus Chlorschwefelsäure und niedrigem chloriertem aliphatischem Kohlenwasserstoff mit einer Geschwindigkeit im Bereich 1,25 bis 1,5 kg/Minute umfasst.

8. Verfahren nach einem der Ansprüche 1 bis 7, das das Aufrechterhalten der Reaktionstemperatur während der Zugabe der Citronensäure und der anschließenden Gasbildung zwischen 10 °C und 15 °C umfasst.

## Revendications

1. Un procédé pour la préparation de 3-oxoglutarates de dialkyle présentant la formule générale (1): 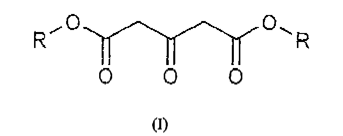 dans laquelle R représente un radical méthyle ou éthyle, par réaction d'acide citrique présentant la formule (II) 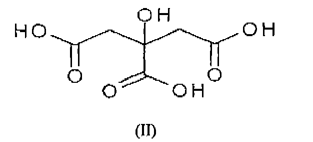 ou le monohydrate en dérivant, avec de l'acide chlorosulfonique dans un hydrocarbure aliphatique inférieur chloré et par réaction de l'intermédiaire ainsi obtenu avec du méthanol ou de l'éthanol, ce procédé comprenant l'utilisation d'un rapport hydrocarbure aliphatique inférieur chloré/acide chlorosulfonique compris entre 0,1/1 et 1/1 en volume et l'addition d'acide citrique au mélange d'acide chlorosulfonique et d'hydrocarbure aliphatique inférieur à raison de 1-2 kg/minute.

2. Un procédé tel que revendiqué dans la revendication 1, qui comprend l'emploi, en tant qu'hydrocarbure aliphatique inférieur, de chlorure de méthylène, de chlorure d'éthylène ou de chloroforme.

3. Un procédé tel que revendiqué dans la revendication 2, qui comprend l'emploi de chlorure de méthylène.

4. Un procédé tel que revendiqué dans la revendication 3, qui comprend l'emploi de chlorure de méthylène et d'acide chlorosulfonique en un rapport volumique compris entre 0,4/1 et 0,6/1.

5. Un procédé tel que revendiqué dans la revendication 4, qui comprend l'emploi de chlorure de méthylène et d'acide chlorosulfonique en un rapport volumique de 0,5/1.

6. Un procédé tel que revendiqué dans une quelconque des revendications 1 à 5, qui comprend l'addition d'acide citrique au mélange d'acide chlorosulfonique et d'hydrocarbure aliphatique inférieur à raison d'environ 1,1 à 1,8 kg/minute.

7. Un procédé tel que revendiqué dans la revendication 6, qui comprend l'addition d'acide citrique au mélange d'acide chlorosulfonique et d'hydrocarbure aliphatique inférieur à raison d'environ 1,25 to 1,5 kg/minute.

8. Un procédé tel que revendiqué dans une quelconque des revendications 1 to 7, qui comprend, pendant l'addition de l'acide citrique et la formation de gaz subséquente, le maintien de la température réactionnelle à une valeur comprise entre 10°C et 15°C,
